# EUROPEAN PATENT SPECIFICATION

(11) **EP 2 435 066 B1**
(45) Date of publication and mention of the grant of the patent: **20.11.2013**
(21) Application number: 10730209.3
(22) Date of filing: 28.05.2010
(51) Int. Cl.: A61K 39/145, C07K 1/20, C07K 1/34, G01N 33/68, C12N 7/00

(54) **ASSAYS FOR INFLUENZA VIRUS HEMAGGLUTININS**
ASSAY FÜR INFLUENZAVIRUS HÄMAGGLUTININE
DOSAGE DES HEMAGGLUTININES DU VIRUS DE LA GRIPPE

(30) Priority: 29.05.2009 US 217405 P
(43) Date of publication of application: 04.04.2012
(73) Proprietor: Novartis AG, 4056 Basel (CH)
(72) Inventor: DORMITZER, Philip, Cambridge, MA 02139 (US); WEN, Yingxia, Cambridge, MA 02139 (US); PALMER, Gene, Cambridge, MA 02139 (US); RINELLA, Paola, I-53100 Siena (IT); WU, Ping, Cambridge, MA 02139 (US)
(74) Representative: Marshall, Cameron John
(86) International application number: PCT/IB2010/001423
(87) International publication number: WO 2010/136896

(56) References cited:
- WO-A2-2007/011904
- US-A- 6 048 537
- US-A1- 2004 086 521
- KAPTEYN J C ET AL: "Haemagglutinin quantification and identification of influenza A&B strains propagated in PER.C6<(>R) cells: A novel RP-HPLC method" VACCINE, ELSEVIER LTD, GB LNKD- DOI:10.1016/J.VACCINE.2006.01.046, vol. 24, no. 16, 12 April 2006 (2006-04-12), pages 3137-3144, XP025151231 ISSN: 0264-410X [retrieved on 2006-04-12]
- KAPTEYN J C ET AL: "HPLC-based quantification of haemagglutinin in the production of egg- and MDCK cell-derived influenza virus seasonal and pandemic vaccines" VACCINE, ELSEVIER LTD, GB LNKD- DOI:10.1016/J.VACCINE.2008.11.113, vol. 27, no. 9, 25 February 2009 (2009-02-25), pages 1468-1477, XP026194443 ISSN: 0264-410X [retrieved on 2009-02-25]
- GENG X ET AL: "Liquid chromatography of recombinant proteins and protein drugs" JOURNAL OF CHROMATOGRAPHY B: BIOMEDICAL SCIENCES & APPLICATIONS, ELSEVIER, AMSTERDAM, NL LNKD- DOI:10.1016/J.JCHROMB.2008.01.041, vol. 866, no. 1-2, 15 April 2008 (2008-04-15), pages 133-153, XP022595824 ISSN: 1570-0232 [retrieved on 2008-02-05]

## Description

### TECHNICAL FIELD

This invention is in the field of assays for influenza virus hemagglutinin e.g. for analyzing vaccines.

### BACKGROUND ART

The standard assay for hemagglutinin (HA) content in inactivated influenza vaccines is based on single radial immunodiffusion ("SRID") [1,2], which was recommended by the WHO in 1978 to replace tests based on agglutination of erythrocytes.

Although the SRID assay is well established, it is slow to perform, has poor dynamic range, considerable variability, and it can take a long time to prepare the required specific anti-HA serum and then to calibrate this serum. Thus people have looked for non-SRID assays for quantifying influenza HA.

One approach has been to use reverse-phase high-performance liquid chromatography (RP-HPLC). For instance, reference 3 discloses a method where HA is reduced in the presence of a detergent, alkylated to protect its sulfhydryl groups, applied to a RP-HPLC column, and then eluted with an ion pairing agent in an organic mobile phase. The inventors complain that previous RP-HPLC methods for influenza antigen purification had poor resolution of the protein peaks of interest, gave low recovery and were not quantitative. By increasing the elution temperature to 50-70°C, however, they were able to increase the recovery and reproducibility of RP-HPLC for influenza HA. Further details of this method are disclosed in ref. 4. See also ref. 5, including work with pandemic strains.

2D-HPLC, coupling size-exclusion HPLC with RP-HPLC, has also been used to characterize influenza vaccine constituents [6]. This method was able to quantify HA but was not performed on formaldehyde-treated antigens. Such treatment irreversibly cross-links HA and so it is uncertain if the 2D-HPLC method is suitable for testing bulk inactivated vaccine antigen.

Size-exclusion HPLC on its own has also been used for HA detection and quantification in inactivated split vaccines, including for pandemic strains [7].

There remains a need for further and improved alternatives to the SRID assays for quantifying HA.

### DISCLOSURE OF THE INVENTION

The invention uses a combination of ultrafiltration (UF) and RP-HPLC to analyze influenza HA. A combination of these two techniques is able to quantify HA and may be more reliable than SRID in determining a vaccine's ability to elicit functional antibodies. Moreover, it can be performed without the delay of waiting for immunochemical SRID reagents, but correlates well with SRID results.

One advantage of SRID is that it allows two forms of HA to be distinguished: immunologically-active HA is detected whereas immunologically-inactive HA (denatured/aggregated/misfolded) is not. Thus SRID measures primarily the "useful" HA in a vaccine. Because HPLC is a denaturing technique, however, this advantage is lost, and previous HPLC-based methods for quantifying influenza HA have been unable to distinguish between the two forms of HA. Thus the previous HPLC-based methods may overestimate the content of "useful" HA, giving vaccines with a lower-than-expected immunological activity. To address this disadvantage, a UF step can be used to remove denatured/aggregated HA, and then RP-HPLC can be used to purify, detect, analyze and/or quantify the remaining HA.

Thus the invention provides a method for purifying influenza virus HA in a sample, comprising steps of: (i) ultrafiltration of the sample using a membrane which retains any denatured or aggregated HA to provide a filtrate; and (ii) RP-HPLC of the filtrate to separate any HA therein from any other components therein. HA separated in step (ii) can then be detected, and this detection may be quantitative, thereby permitting calculation of the amount of active HA in the original sample. After such calculation, the sample (or, more usually, bulk material from which the sample was taken) can then be diluted to give material having a desired HA concentration. This material can be used for vaccine manufacture.

The invention also provides, in a process for purifying influenza virus HA in a sample by RP-HPLC, the improvement consisting of subjecting the sample to ultrafiltration prior to RP-HPLC.

Also disclosed is bulk antigen containing influenza HA, wherein a sample of the bulk material has been analyzed by a method of the invention.

In comparison to the methods of references 3 to 5, the invention does not require the step in which sulfhydryls are derivatized and does not require high elution temperatures. Either or both of these features may be used with the invention if desired (*e.g.* a high elution temperature can be useful), but they are not required.

### Ultrafiltration

UP is a membrane filtration technique in which hydrostatic pressure forces a liquid against a semipermeable membrane. Suspended solids and solutes of high molecular weight are retained, while water and low molecular weight solutes can pass through the membrane. The molecular weight cut-off (MWCO) of a UF membrane determines which solutes can pass through the membrane (*i.e.* into the filtrate) and which are retained (*i.e.* in the retentate).

For the invention, a membrane is selected which retains any denatured/aggregated HA while permitting the passage of any active HA. A 300kDa cut-off is convenient for this separation, but other cut-offs can also be used. Influenza HA glycoprotein is about 75kDa as a monomer, but these assemble in the virion as a homotrimer of about 230kDa. The precise molecular weight varies according to virus strain and glycosylation, but a UF membrane cut-off can easily be selected to permit the passage of desired monomers and/or trimers while retaining higher molecular weight aggregates. If HA fragments are to be purified (see below) then the cut-off can be decreased accordingly.

UF can be operated in various formats *e.g.* cross-flow (tangential flow; TFUF) or normal flow (dead ended). Although either format can be used, normal flow is more convenient for analytical methods of the invention. A UF membrane inside a centrifugation tube may be used (*e.g*. a centrifugal UF concentrator).

Various types of UF membrane are available, such as spiral wound modules (large consecutive layers of membrane and support material rolled up around a tube), tubular membranes (sample flows through a core and fltrate passes outwards into a tubular housing), hollow fiber membranes (sample flows through open cores of fibers and filtrate is collected in the cartridge area surrounding the fibers), and vertical membranes (sample is loaded info a tube and flows through a membrane which is vertical, in a plane substantially parallel to the tube's long axis). Any of these membrane arrangements can be used.

UF can be performed under pressure. Typically the sample is pressurized by pumping while the filtrate is left at atmospheric pressure.

Various materials are used in UF membranes. The method can conveniently use a polyethersulfone membrane.

### RP-HPLC

UF removes denatured/aggregated HA from the original sample, whereas active HA passes into the filtrate. This filtrate is then subjected to RP-HPLC to separate the HA from any other proteins in the filtrate (*e.g*. from other influenza virus antigens, or from non-influenza proteins). This separation results in purification of the HA and the purified material can be analyzed *e.g*. it can be quantified.

HPLC is a form of chromatography which applies a liquid (mobile phase, such as a solvent) to a chromatographic column (stationary phase), with retention on the column depending on the interactions between the stationary phase and components present in a sample. A pump moves the liquid phase through the column and, as conditions change, different molecules can elute from the column at different times. RP-HPLC has a non-polar stationary phase and an aqueous, moderately polar mobile phase. RP-HPLC retention times can generally be increased by increasing the proportion of water in the mobile phase (thereby making the affinity of a hydrophobic analyte for a hydrophobic stationary phase stronger relative to the now more hydrophilic mobile phase); conversely they can be decreased by increasing the proportion of non-polar or less-polar organic solvent (*e.g*. methanol, acetonitrile).

The RP-HPLC stage separates any HA in the UF-treated sample from other proteins. Thus the RP-HPLC column and elution conditions are selected such that the HA can be resolved from these other proteins. The ability of RP-HPLC to achieve this resolution is already known from *e.g*. ref. 4.

Various forms of RP-HPLC are available. The invention can conveniently be performed on a column of 10µm polystyrenedivinylbenzene (PSDVB) particles with a 4000 Å pore size, but other support materials (*e.g*. other hydrophobic polymers, such as *n*-alkyl hydrophobic chains of octadecyl, decyl or butyl covalently bonded to silanol groups in silica), particle sizes (*e.g*. 3-50µm) and pore sizes (*e.g*. between 250-5000 Å) can be used, and the properties of PSDVB can be changed by changing the ratio of PS and DVB during copolymerization, or β-derivatisation (*e.g*. sulfoacylation). Suitable RP-HPLC supports can readily be selected based on their ability to retain and elute HA and to separate it from other materials which are present in a sample. Supports with beads having two pore classes can be used: large "throughpores" which allow convection flow to occur through the particles themselves, quickly carrying sample molecules to short "diffusive" pores inside. This pore arrangement reduces the distance over which diffusion needs to occur and reduces the time required for sample molecules to interact with binding sites. Thus diffusion can be non-limiting and flow rates can be increased (*e.g*. 1000-5000 cm/hour) without compromising resolution or capacity.

Various elution buffers can be used e.g. using an acetonitrile gradient. Suitable flow rates can readily be selected e.g. between 0.1 and 5ml/min (e.g. between 0.5 and 1.5ml/min, or about 0.8ml/min). Elution can take place at room temperature but, as described in reference 3, elution in the range of 50-70°C is helpful *e.g*. between 55-65°G, or at about 60°C.

The RP-HPLC eluate can be monitored (*e.g*. for UV absorbance at about 214nm, or for intrinsic fluorescence using excitation at about 290nm and emission at about 335nm) to detect any HA in the UF-treated sample. The area under the HA peak on a HPLC elution chromatogram can be used to quantify the HA. Thus the method permits calculation of the amount of HA in the UF-treated sample, and therefore the amount of active HA in the pre-UF sample. By using samples of known volume, the amounts of HA determined by these methods can then be used to calculate the HA concentration in the original material from which the sample was taken *e.g*. in a bulk antigen preparation, or in an individual vaccine dose.

The UF filtrate may feed directly into the RP-HPLC (*e.g*. in an in-line setup) or may be collected and then introduced separately. In some embodiments the filtrate is treated prior to RP-HPLC. For instance, reference 3 performs an alkylation step prior to RP-HPLC, and this step can be performed with the present invention although it is not necessary (and thus is not preferred). One useful step between UF and RP-HPLC is to treat the filtrate with detergent. Addition of detergent can solubilize any filtrate HA which is not monomeric *e.g*. which is in the form of rosettes in a lipid bilayer. For this reason the detergent treatment occurs after UF but before RP-HPLC, as treatment before UF could solubilise non-native HA (denatured/aggregated), which would let it pass through the UF filter and thus give misleading results.

Suitable solubilizing detergents may be ionic, non-ionic or zwitterionic, and include, but are not limited to: deoxycholate; tri-N-butyl phosphate; cetyltrimethylammonium bromide; Tergitol NP9; alkylglycosides; alkylthioglycosides; acyl sugars; sulphobetaines; betains; polyoxyethylenealkylethers; N;N-dialkyl-Glucamides; Hecameg; alkyl-phenoxy-polyethoxyethanols; sarcosyl; myristyltrimethylammonium salts; lipofectin; lipofectamine; and DOT-MA; the octyl- or nonylphenoxy polyoxyethanols (*e.g*. the Triton surfactants; such as Triton X100 or Triton N101); polyoxyethylene sorbitan esters (the Tween surfactants, such as polysorbate 80); polyoxyethylene ethers; polyoxyethlene esters; the amphoteric 'Zwittergent' detergents such as 'Zwittergent 3-14'™ (*n*-Tetradecyl-N,N-dimethyl-3-ammonio-1-propanesulfonate; CAS 14933-09-6; 'TDAPS'); alkylene glycol monododecyl ethers, such as octaethylene glycol monododecyl ether (`C12E8'); *etc.* These are added at a level high enough to solubilize HA (*e.g*. up to 5% (v/v), but typically about 1%) but not so high as to interfere with subsequent analysis. Two specific detergents of interest are Zwittergent 3-14™ and C12E8. For instance, treatment with 1% Zwittergent for 30 minutes at room temperature provides good analytical results.

### The sample

The sample which is subjected to UF and RP-HPLC contains (or is at least suspected to contain) influenza virus HA. SRID is often performed on material containing HA from several strains (*e.g*. trivalent material). The invention can be used with multivalent material but cannot separately quantify the antigen from each strain unless the different HAs can be resolved by the HPLC column. In some circumstances, though, it is adequate to quantify total HA in a multivalent mixture without having to know the contribution from each strain. In general, however, a sample contains HA from only one influenza virus strain *i.e.* it is monovalent.

The invention can be used with various types of sample. It can be used to analyze a final vaccine, but as the analytical method is destructive this will usually be a single vaccine from a batch, with analytical results indicating properties of the batch. The sample may have been taken from bulk vaccine matenal, with analytical results indicating properties of the bulk as a whole. In other embodiments the sample may even be virus-containing fluids *e.g.* it may be virus-containing harvest fluids from a cell culture or from eggs. The sample may thus be starting material, final vaccine material, or any manufacturing intermediate between virus culture and final vaccine.

The sample will typically include HA from influenza virions but, as an alternative, it may include HA which was expressed in a recombinant host (*e.g*. in an insect cell line using a baculovirus vector) and purified [8,9,10] or may be in the form of virus-like particles (VLPs; *e.g*. see references 11 and 12). In general, however, antigens will be from virions and so the sample may, in addition to HA, include other influenza virus proteins (*e.g.* PB1, PB2, PA, NP, NA, M1, M2, NS1 and/or NS2 proteins), and may also include influenza virus lipids.

Virion-derived influenza virus antigens are based either on live virus or on inactivated virus (*e.g.* see chapters 17 & 18 of reference 13). Although the invention can be used to determine HA levels from live virus, dosing of live vaccines is based on median tissue culture infectious dose rather than HA content, and so the invention will usually be used to determine HA levels in inactivated material.

Inactivated vaccines may be based on whole virions, 'split' virions, purified surface antigens (including HA and, usually, also including neuraminidase) or virosomes (nucleic acid free viral-like liposomal particles [14]). The invention can be used with all such vaccines. The BEGRIVAC™, FLUARIX™, PREPANDRIX™, FLUZONE™ and FLUSHIELD™ products are split vaccines. The FLUVIRIN™, AGRIPPAL™, FLUAD™ and INFLUVAC™ products are surface antigen vaccines. The INFLEXAL V™ and INVAVAC™ products are virosome vaccines. The invention is most useful for measuring HA content in split and surface antigen vaccines.

The invention can be used with HA from any influenza virus type, including both influenza A virus and influenza B virus.

For influenza A viruses the invention can be used with HA from any known HA subtype (H1, H2, H3, H4, H5, H6, H7, H8, H9, H10, H11, H12, H13, H14, H15 or H16), and it is particularly useful with H1, H3 and H5 strains. The strain may have any of NA subtypes N1, N2, N3, N4, N5, N6, N7, N8 or N9. For example, the invention can be used to analyze material from a H1N1 strain, a H3N2 strain, a H5N1 strain, *etc.* The invention is particularly useful for H1 strains, and in some embodiments the strain is a H1 strain (*e.g*. a H1N1 strain) with a HA that is more closely related to SEQ ID NO: 1 (HA1 from A/California/04/2009) than to SEQ ID NO: 2 (HA1 from A/Chile/1/1983) *i.e*. it has a higher degree sequence identity when compared to SEQ ID NO: 1 than to SEQ ID NO: 2 using the same comparison algorithm and parameters. In other embodiments a H1 HA is more closely related to SEQ ID NO: 2 than to SEQ ID NO: 1.

For influenza B viruses the invention can be used with HA from a B/Victoria/2/87-like strain or a B/Yamagata/16/88-like strain. These two groups of strains are usually distinguished antigenically, but differences in amino acid sequences have also been described for distinguishing the two lineages *e.g*. B/Yamagata/16/88-like strains often (but not always) have HA proteins with deletions at amino acid residue 164, numbered relative to the 'Lee40' HA sequence [15].

HA which is analyzed by methods of the invention may be full-length precursor HA, known as HA0. In some embodiments, however, the HA is a fragment of HA0. HA0 can be fragmented by serine proteases, such as trypsin, to yield a N-terminal fragment (HA1) and a C-terminal fragment (HA2), which can remain joined by a disulfide bridge. Thus the methods of the invention may purify HA0, HA1 or HA2, and may involve detection and analysis of only one such fragment (*e.g*. of HA1). Other HA fragments which might be purified and analyzed include, for instance, the fragment released by bromelain treatment, or fragments obtained by treatment with both bromelain and trypsin. Typically the methods will purify HA1, and thus at least the RP-HPLC step should be performed under reducing conditions (*e.g*. using DTT) to ensure HA1 and HA2 are separated, and optionally may involve a pre-purification digestion step (*e.g*. using trypsin) to ensure full cleavage of HA1 from HA2 (but this digestion is often unnecessary, and the presence of any full-length HA0 can easily be tested to determine if digestion would be useful). Reference 4 reports that HA1 is well separated from HA2 and other vaccine components by RP-HPLC.

HA in the sample may include a hyper-basic region around the HA1/HA2 cleavage site, but in other embodiments this region is absent.

HA in the sample may have a binding preference for oligosaccharides with a Sia(α2,6)Gal terminal disaccharide compared to oligosaccharides with a Sia(α2,3)Gal terminal disaccharide, or *vice versa*, or it may show no such preference. Assays for this preference are discussed in reference 16. Human influenza viruses bind to receptor oligosaccharides having a Sia(α2,6)Gal terminal disaccharide (sialic acid linked α-2,6 to galactose), but eggs and Vero cells have receptor oligosaccharides with a Sia(α2,3)Gal terminal disaccharide.

In some embodiments the HA has a different glycosylation pattern from the patterns seen in egg-derived viruses. Thus the HA may include glycoforms that are not seen in chicken eggs. Useful HA includes canine glycoforms *e.g*. the virus may have been grown in MDCK cells. Other useful HA includes human glycoforms *e.g*. the virus may have been grown in PER.C6 cells. Other useful HA includes simian glycoforms *e.g*. the virus may have been grown in Vero cells. These cell lines are widely available *e.g*. from the American Type Cell Culture (ATCC) collection, from the Coriell Cell Repositories, or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. One suitable MDCK cell line is 'MDCK 33016', deposited as DSM ACC 2219 [17].

HA in the sample may be from a wild-type virus or from a reassortant virus, particularly for influenza A virus. A reassortant virus may have been obtained by reverse genetics techniques. Thus a sample may include HA from one virus strain but other influenza antigens (*e.g*. PB1, PB2, PA, NP, M1, M2, NS1 and/or NS2 proteins) from a different strain *e.g*. from A/PR/8/34, from A/AA/6/60, or from A/WSN/33.

The HA concentration in a sample will usually be between 0.1µg/ml and 10mg/ml *e.g*. between 1µg/ml and 1mg/ml, or between 10µg/ml and 100µg/ml. In some embodiments the concentration is about 30µg/ml, about 15µg/ml or about 7.5µg/ml.

The sample may include serum components but is preferably free from such components.

The sample may include egg proteins (*e.g*. ovalbumin and ovomucoid) and/or chicken DNA, but in some embodiments is free from these components *e.g*. when virus was grown in cell culture.

The sample may include DNA *e.g*. chicken DNA or mammalian DNA (*e.g*. derived from MDCK cells, Vero cells, PER.C6 cells, *etc*.). Ideally, however, a sample contains less than 600ng of DNA per mg of HA (preferably less than 60ng, and more preferably less than 6ng).

The sample preferably includes no viral proteins except for influenza virus proteins.

The sample may include detergent *e.g*. a polyoxyethylene sorbitan ester surfactant (known as 'Tweens' *e.g*. polysorbate 80), an octoxynol (such as octoxynol-9 (Triton X-100) or -10, or t-octylphenoxypolyethoxyethanol), a cetyl trimethyl ammonium bromide ('CTAB'), or sodium deoxycholate, particularly for a split or surface antigen vaccine sample. The detergent may be present only at trace amounts *e.g*. residual from antigen manufacture. Other sample components in trace amounts could be antibiotics (*e.g*. neomycin, kanamycin, polymyxin B).

### Downstream steps

Methods of the invention permit the measurement of HA concentration in material of interest. This material, particularly a bulk vaccine, can then be diluted to give a desired final HA concentration. Thus a method of the invention may include a further step of diluting a vaccine based on the results of HA purification, and the invention provides a method for analyzing a vaccine comprising steps of: (a) purifying HA in the vaccine, or in a sample thereof, by a method as disclosed herein; and (b) using the results of step (a) to calculate the HA concentration in the vaccine. The invention also provides a method for providing a bulk vaccine with a desired HA concentration, comprising said steps (a) and (b), and a further step (c) using the results of step (b) to dilute the bulk vaccine to give the desired HA concentration: Unit doses of the diluted bulk vaccine can then be extracted, and SQ the invention provides a method for providing a vaccine for patient use, comprising said steps (a) to (c), and a further step (d) extracting one or more unit doses of vaccine from the diluted bulk, each unit dose having a desired HA content. The extracted material described can be placed into a container *e.g*. a vial or syringe.

Diluted bulk can be mixed with other components, such as an adjuvant, prior to unit dose extraction. Thus the invention provides a method for providing a bulk adjuvanted vaccine comprising said steps (a) to (c), and a further step (d) mixing the diluted bulk vaccine with an adjuvant. The invention also provides a method for providing an adjuvanted vaccine for patient use, comprising said steps (a) to (d), and a further step (e) extracting one or more unit doses of vaccine from the diluted bult, each unit dose having a desired HA content. The extracted material described can be placed into a container *e.g*. a vial or syringe.

As an alternative to mixing an extracted dose with an adjuvant, the extracted dose may instead be packaged as a first kit component in combination with a second kit component, wherein the second kit component is a vaccine adjuvant. The two kit components described can be combined at the time of use to give an adjuvanted vaccine. The kit allows the adjuvant and the antigen to be kept separately until the time of use (*e.g*. as in the PREPANDRIX™ product). The components are physically separate from each other within the kit, and this separation can be achieved in various ways. For instance, the two components may be in two separate containers, such as vials. The contents of the two vials can then be mixed *e.g*. by removing the contents of one vial and adding them to the other vial, or by separately removing the contents of both vials and mixing them in a third container. In one arrangement, one of the kit components is in a syringe and the other is in a container such as a vial. The syringe can be used (*e.g.* with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration. In another arrangement described, the two kit components are held together but separately in the same syringe *e.g*. a dual-chamber syringe [18]. When the syringe is actuated (*e.g*. during administration to a patient) the contents of the two chambers are mixed. This arrangement avoids the need for a separate mixing step at the time of use.

Whether antigen and adjuvant are mixed during manufacture or at the time of delivery to a patient, antigen will generally be aqueous and so the mixing step involves mixing two liquids. The volume ratio of the two liquids for mixing can vary (*e.g*. between 5:1 and 1:5) but is generally about 1:1. Thus two kit components described may include substantially the same volume of liquid as each other.

The adjuvant can enhance the immune responses (humoral and/or cellular) elicited in a patient who receives the composition. A typical adjuvant for this purpose is an oil-in-water emulsion. Various suitable emulsions are known, and they typically include at least one oil and at least one surfactant, with the oil(s) and surfactant(s) being biodegradable (metabolisable) and biocompatible. The oil droplets in the emulsion are generally less than 5µm in diameter, and advantageously the emulsion comprises oil droplets with a sub-micron diameter, with these small sizes being achieved with a microfluidiser to provide stable emulsions. Droplets with a size less than 220nm are preferred as they can be subjected to filter sterilization.

The emulsion can include oils from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolisable and may therefore be used in the practice of this invention. The procedures for separation, purification, saponification and other means necessary for obtaining pure oils from animal sources are well known in the art. Most fish contain metabolisable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoid known as squalene, 2,6,10,15,19,23-hexamethyl-2,6,10,14,18,22-tetracosahexaene. Other preferred oils are the tocopherols, including DL-α-tocopherol. Emulsions comprising squalene are particularly preferred. Mixtures of oils can be used.

Surfactants can be classified by their 'HLB' (hydrophile/lipophile balance). Preferred surfactants of the invention have a HLB of at least 10, preferably at least 15, and more preferably at least 16. Examples of suitable surfactants include, but are not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate; polysorbate 80), Span 85 (sorbitan trioleate), lecithin and Triton X-100. Inclusion of polysorbate 80 is preferred.

Mixtures of surfactants can be used *e.g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [19-21], as described in more detail in Chapter 10 of ref. 22 and chapter 12 of ref. 23. The MF59 emulsion advantageously includes citrate ions *e.g*. 10mM sodium citrate buffer.
- An emulsion comprising squalene, an α-tocopherol, and polysorbate 80. These emulsions may have from 2 to 10% squalene, from 2 to 10% tocopherol and from 0.3 to 3% Tween 80, and the weight ratio of squalene:tocopherol is preferably ≤1 (*e.g*. 0.90) as this provides a more stable emulsion. Squalene and Tween 80 may be present at a volume ratio of about 5:2, or at a weight ratio of about 11:5. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.
- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate (*e.g*. polysorbate 80), a Triton detergent (*e.g*. Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [24] (0.05-1% Thr-MDP, 5% squalane, 2.5% Pluronic L121 and 0.2% polysorbate 80). It can also be used without the Thr-MDP, as in the "AF" adjuvant [25] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [26]. The emulsion may also include one or more of: alditol; a cryoprotective agent (*e.g*. a sugar, such as dodecylmaltoside and/or sucrose); and/or an alkylpolyglycoside. Such emulsions may be lyophilized.
- An emulsion having from 0.5-50% of an oil, 0.1-10% of a phospholipid, and 0.05-5% of a non-ionic surfactant. As described in reference 27, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 28, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [29].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [29].
- An emulsion in which a saponin (*e.g*. QuilA or QS21) and a sterol (*e.g*. a cholesterol) are associated as helical micelles [30].

The importance of measuring HA content in vaccines, and of diluting bulk vaccines to a desired HA content, arises because inactivated influenza vaccines are standardized by their HA levels. Current vaccines typically contain about 30µg/ml of HA per strain, although lower doses are also used *e.g*. for children, or in emergency situations. Fractional doses such as ½ (*i.e.* 15µg/ml HA, as in FOCETRIA™), ¼ (*i.e*. 7.5µg/ml, as in PREPANDRIX™ when administered) and ⅛ have been used [31,32], as have higher doses (*e.g*. 3x or 9x doses [33,34]).Thus dilution may be performed to give a HA concentration between 0.1 and 200µg/ml per influenza virus strain, preferably between 1 and 150µg/ml *e.g*. 1-90µg/ml, 1-20µml, 0.1-15µg/ml, 0.1-10µg/ml, 0.1-7.5µg/ml, 0.5-5µg/ml, 3.75-15µg/ml *etc.* Particular post-dilution concentrations include *e.g.* about 90µg/ml, about 45µg/ml, about 30 µg/ml, about 15 µg/ml, about 10 µg/ml, about 7.5 µg/ml, about 5 µg/ml, about 3.8 µg/ml, about 3.75 µg/ml, about 1.9 µg/ml, about 1.5 µg/ml, *etc..* Lower concentrations (*e.g*. <30µg/ml) are most useful when an adjuvant is present in the vaccine. Although concentrations as high as 180µg/ml have been used in some studies (*e.g*. reference 35), compositions described will usually have a HA concentration of ≤30µg/ml.

### Pharmaceutical compositions

The described HA-containing compositions for administration to patients are pharmaceutically acceptable. They usually include components in addition to the HA and optional adjuvant *e.g*. they typically include one or more pharmaceutical carrier(s) and/or excipient(s). A thorough discussion of such components is available in reference 36.

Pharmaceutical compositions will generally be in aqueous form (*e.g*. for injection) but solid dosage forms can also be used, and are known for influenza vaccination.

A pharmaceutical composition may include a preservative such as thiomersal (*e.g* at 10µg/ml) or 2-phenoxyethanol. It is preferred, however, that a vaccine should be substantially free from (*i.e.* less than 5µg/ml) mercurial material *e.g*. thiomersal-free [37]. Vaccines containing no mercury are more preferred. Preservative-free vaccines are particularly preferred.

To control tonicity, an aqueous pharmaceutical composition may include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Aqueous pharmaceutical compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [38], but keeping osmolality in this range is nevertheless preferred.

Pharmaceutical compositions may include one or more buffers. Typical buffers include: a phosphate buffer; a Tris buffer; a borate buffer; a succinate buffer; a histidine buffer; or a citrate buffer. Buffers will typically be included in the 5-20mM range. The buffer may be in an emulsion's aqueous phase.

The pH of a pharmaceutical composition will generally be between 5.0 and 8.1, and more typically between 6.0 and 8.0 *e.g.* 6.5 and 7.5, or between 7.0 and 7.8. A process of the invention may therefore include a step of adjusting the pH of a bulk prior to dose extraction or packaging.

A pharmaceutical composition is preferably sterile. A pharmaceutical composition is preferably gluten free.

Preferred pharmaceutical compositions have a low endotoxin content *e.g*. less than 1 IU/ml, and preferably less than 0.5 IU/ml. The international unit for endotoxin measurement is well known and can be calculated for a sample by, for instance, comparison to an international standard [39,40], such as the *2nd International Standard* (Code 94/580 - IS) available from the NIBSC. Current vaccines prepared from virus grown in eggs have endotoxin levels in the region of 0.5-5 IU/ml.

A pharmaceutical composition may be free from antibiotics (*e.g*. neomycin, kanamycin, polymyxin B).

A pharmaceutical composition may include material for a single immunisation, or may include material for multiple immunisations (*i.e*. a 'multidose' composition, in which case an extracted unit dose contains enough material for more than one patient dose). Multidose arrangements usually include a preservative in the vaccine. To avoid this need, a vaccine may be contained in a container having an aseptic adaptor for removal of material.

Influenza vaccines are typically administered by intramuscular injection in a dosage volume of about 0.5ml, although a half dose (*i.e*. about 0.25ml) may be administered to children, and unit doses will be selected accordingly *e.g*. a unit dose to give a 0.5ml dose for administration to a single patient. Lower dosage volumes may be used for *e.g*. a 0.1ml volume is useful for intradermal injection.

### Packaging of compositions or kit components

Processes of the invention can include a step in which vaccine is placed into a container, and in particular into a container for distribution for use by physicians. This step will usually involve extraction of material from a bulk and its insertion into the container.

Suitable containers for aqueous vaccines include vials, nasal sprays and disposable syringes, which should be sterile.

Where a composition/component is located in a vial, the vial is preferably made of a glass or plastic material. The vial is preferably sterilized before the composition is added to it. To avoid problems with latex-sensitive patients, vials may be sealed with a latex-free stopper, and the absence of latex in all packaging material is preferred. The vial may include a single dose of vaccine, or it may include more than one dose (a 'multidose' vial) *e.g*. 10 doses. Preferred vials are made of colorless glass.

A vial can have a cap (*e.g*. a Luer lock) adapted such that a syringe can be inserted therein. A vial may have a cap that permits aseptic removal of its contents, particularly for multidose vials.

Where a composition/component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Useful syringes are those marketed under the trade name "Tip-Lok"™. Other useful syringes include those suitable for intradermal administration *e.g*. a microinjection device with a needle about 1.5mm long.

Containers may be marked to show a half-dose volume *e.g*. to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A composition may be combined (*e.g*. in the same box) with a leaflet including details of the vaccine *e.g.* instructions for administration, details of the antigens within the vaccine, *etc.* The instructions may also contain warnings *e.g*. to keep a solution of adrenaline readily available in case of anaphylactic reaction following vaccination, *etc.*

### Methods of treatment, and administration of the vaccine

The described compositions are suitable for administration to animals, such as humans, and described is herein a method of raising an immune response in an animal, comprising the step of administering a composition described herein to the patient.

Described is herein a kit or composition for use as a medicament *e.g*. for raising an immune response in an animal. Described is herein the use of a composition in the manufacture of a medicament for raising an immune response in an animal.

Immune response raised by methods and uses described herein will generally include an antibody response, preferably a protective antibody response. Methods for assessing antibody responses, neutralising capability and protection after influenza virus vaccination are well known in the art. Human studies have shown that antibody titers against HA of human influenza virus are correlated with protection (a serum sample hemagglutination-inhibition titer of about 30-40 gives around 50% protection from infection by a homologous virus) [41]. Antibody responses are typically measured by hemagglutination inhibition, by microneutralisation, by single radial immunodiffusion (SRID), and/or by single radial hemolysis (SRH). These assay techniques are well known in the art.

Influenza vaccines can be administered in various ways. The most preferred immunisation route is by intramuscular injection (*e.g*. into the arm or leg), but other available routes include subcutaneous injection, intranasal [42-44], intradermal [45,46], oral [47], transcutaneous, transdermal [48], *etc.* Intradermal and intranasal routes are attractive. Intradermal administration may involve a microinjection device *e.g*. with a needle about 1.5mm long.

The described vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g*. ≥50 years old, ≥60 years old, and preferably ≥65 years), the young (*e.g*. ≤5 years old), hospitalised patients, healthcare workers, armed service and military personnel, pregnant women, the chronically ill, immunodeficient patients, patients who have taken an antiviral compound (*e.g*. an oseltamivir or zananuvir compound; see below) in the 7 days prior to receiving the vaccine, people with egg allergies and people travelling abroad. The vaccines are not suitable solely for these groups, however, and may be used more generally in a population.

Preferred compositions satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients. The criteria apply for each strain in a vaccine.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc.* Administration of more than one dose (typically two doses) is particularly useful in immunologically naïve patients *e.g*. for people who have never received an influenza vaccine before, or for vaccinating against a new HA subtype. Multiple doses will typically be administered at least 1 week apart (*e.g*. about 2 weeks, about 3 weeks, about 4 weeks, about 6 weeks, about 8 weeks, about 12 weeks, about 16 weeks, *etc.*).

The described vaccines produced by the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, the described vaccines may be administered to patients at substantially the same time as (*e.g*. during the same medical consultation or visit to a healthcare professional) an antiviral compound, and in particular an antiviral compound active against influenza virus (*e.g*. an oseltamivir and/or a zanamivir).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x is optional and means, for example, *x*±10%.

"GI" numbering is used above. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g*. for correction, or to add more annotation or information) then it receives a new GI number. Thus the sequence associated with a given GI number is never changed.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production *e.g*. as in Ph Eur general chapter 5.2.3.

Identity between polypeptide sequences is preferably determined by the Smith-Waterman homology search algorithm as implemented in the MPSRCH program (Oxford Molecular), using an affine gap search with parameters *gap open penalty*=*12* and *gap extension penalty*=*1*.

### MODES FOR CARRYING OUT THE INVENTION

RP-HPLC was tested as a way of quantifying influenza HA in monovalent influenza virus antigen bulk ("monobulks"). RP-HPLC was found to give good quantification of HA when the monobulks had high specific purity and stable HA, and the quantitative results closely matched standard SRID results. In circumstances when the vaccine included significant quantities of denatured HA, however, the RP-HPLC method no longer matched the SRID assay.

For example, the following table shows results from four A/H3N2 monobulks. Total protein concentration (µg/ml) was assessed by BCA, and then HA concentration (µg/ml) was assayed by both SRID (standard protocol) and RP-HPLC. The RP-HPLC was performed on a Poros™ R1/10 column, 2.1 mm x 100 mm, operated at 60°C with a flow rate of 0.8ml/min. The mobile phases were: (A) 0.1% TFA, 5% acetonitrile in water; and (B) 0.1% TFA in 100% acetonitrile (solvent B), changing from an A/B mixture of 20%/80% to 0%/100% over 6.5 minutes.

Prior to RP-HPLC, HA in the monobulks was found to be cleaved already into HA1 and HA2 and addition of DTT (final concentration of 25 mM, followed by heating for 10 minutes at 90°C) ensured that these were separated. Samples were diluted with PBS into the calibrated range. The method was calibrated using a NIBSC reference sample of HA (unfiltered).

RP-HPLC could easily resolve the HA1 peak and so this was used for quantification.

Results were as follows:

| **Monobulk** | **Total protein** | **HA (SRID)** | **HA (HPLC)** |
|---|---|---|---|
| A | 1256 | 336 | 358 |
| B | 645 | 192 | 266 |
| C | 728 | 134 | 298 |
| D | 830 | 68 | 302 |

Thus HPLC agreed well with SRID for monobulk A, but not for B, C or D. These three monobulks were found to be of poor quality. Thus the RP-HPLC method had to be improved so that it would give good results regardless of the monobulk's quality.

A pre-HPLC treatment step was introduced using ultrafiltration. A VivaSpin™ 300kD MWCO spin column, 500µl volume with PES membrane, was found to trap aggregated HA in monobulk and the filtrate would be easily assayed by RP-HPLC to give results in good agreement with SRID.

Prior to the ultrafiltration, samples were diluted (if necessary) to give a protein concentration <100µg/ml. Between the ultrafiltration and RP-HPLC steps the filtrate was incubated with a final concentration of 1% Zwittergent (9 parts filtrate + 1 part 10% Zwittergent 10%) for 30 minutes at room temperature. RP-HPLC was performed as before.

The following table shows HA content measured by SRID or RP-HPLC with the pre-treatment UF step, in comparison to HA content measured without the UF step:

| **Monobulk** | **Total protein** | **HA (SRID)** | **HA (HPLC) No filtration** | **HA (HPLC) Pre-UF** |
|---|---|---|---|---|
| E | 605 | 131 | 187 | 129 |
| F | 695 | 106 | 270 | 113 |

Thus the pre-treatment UF step brings the RP-HPLC results into close correspondence with the SRID results.

Preliminary experiments were also performed with an A/H1N1 strain. Although the method could successfully purify HA1, and the UF pre-treatment removed aggregates, the antigen content as measured by RP-HPLC did not correlate so well with SRID. When the relevant antigen standard was tested, however, its content differed >2-fold from its reference value. Thus the antigen standard seems to be flawed and so the results of these experiments are unreliable.

In conclusion, RP-HPLC systematically overestimates HA content, especially in monobulks with significant amounts of denatured HA. After a pre-treatment UF step, however, the values agree very closely with SRID.

### REFERENCES

[1] Williams (1993) Vet Microbiol 37:253-262.
[2] Fitzgerald & Needy (1986) Dev Biol Stand 64:73-79.
[3] WO2005/090390.
[4] Kapteyn et al. (2006) Vaccine 24:3137-44.
[5] Kapteyn et al. (2009) Vaccine 27:1468-77.
[6] García-Cañas et al. (2007) Anal. Chem. 79(8): 3164-72.
[7] Michaelides (2008) Studies by Undergraduate Researchers at Guelph 1(2):7-19.
[8] WO96/37624.
[9] WO98/46262.
[10] WO95/18861.
[11] Bright et al. (2008) PLoS ONE 3:e1501.
[12] Crevar & Ross (2008) Virology Journal 5:131.
[13] Vaccines. (eds. Plotkin & Orenstein). 4th edition, 2004, ISBN: 0-7216-9688-0.
[14] Huckriede et al. (2003) Methods Enzymol 373:74-91.
[15] GenBank sequence GI:325176.
[16] WO2008/032219.
[17] WO97/37000.
[18] WO2008/001221.
[19] WO90/14837.
[20] Podda & Del Giudice (2003) Expert Rev Vaccines 2:197-203.
[21] Podda (2001) Vaccine 19: 2673-2680.
[22] Vaccine Design: The Subunit and Adjuvant Approach (eds. Powell & Newman) Plenum Press 1995 (ISBN 0-306-44867-X).
*[23]* Vaccine Adjuvants: Preparation Methods and Research Protocols (Volume 42 of Methods in Molecular Medicine series). ISBN: 1-59259-083-7. Ed. O'Hagan.
[24] Allison & Byars (1992) Res Immunol 143:519-25.
[25] Hariharan et al. (1995) Cancer Res 55:3486-9.
[26] US-2007/014805.
[27] WO95/11700.
[28] US patent 6,080,725.
[29] WO2006/113373.
[30] WO2005/097181.
[31] WO01/22992.
[32] Hehme et al. (2004) Virus Res. 103(1-2):163-71.
[33] Treanor et al. (1996) J Infect Dis 173:1467-70.
[34] Keitel et al. (1996) Clin Diagn Lab Immunol 3:507-10.
[35] Zangwill et al. (2008) J Infect Dis. 197(4):580-3.
[36] Gennaro (2000) Remington: The Science and Practice of Pharmacy. 20th edition, ISBN: 0683306472.
[37] Banzhoff (2000) Immunology Letters 71:91-96*.*
[38] Nony et al. (2001) Vaccine 27:3645-51.
[39] Poole & Mussett (1989) J Biol Stand 17:161-71.
[40] Poole et al. (1997) J. Endotoxin Res 4:221-31
[41] Potter & Oxford (1979) Br Med Bull 35: 69-75.
[42] Greenbaum et al. (2004) Vaccine 22:2566-77.
[43] Zurbriggen et al. (2003) Expert Rev Vaccines 2:295-304.
[44] Piascik (2003) J Am Pharm Assoc (Wash DC). 43:728-30.
[45] Halperin et al. (1979) Am J Public Health 69:1247-50.
[46] Herbert et al. (1979) J Infect Dis 140:234-8.
[47] Mann et al. (2004) Vaccine 22:2425-9.
[48] Chen et al. (2003) Vaccine 21:2830-6.

## Claims

1. A method for purifying influenza virus HA in a sample, comprising steps of: (i) ultrafiltration of the sample using a membrane which retains any denatured or aggregated HA to provide a filtrate; and (ii) RP-HPLC of the filtrate to separate any HA therein from any other components therein.

2. The method of claim 1, wherein step (i) uses an ultrafiltration membrane with a 300kDa cut-off.

3. The method of any preceding claim, wherein step (ii) uses a RP-HPLC support of polystyrenedivinylbenzene particles with a pore size between 500-5000 Å.

4. The method of any preceding claim, wherein the sample contains HA from only one influenza virus strain.

5. The method of any preceding claim, wherein the HA comprises:
(a) influenza A virus HA, for example wherein the HA is H1, H3 or H5 HA, and wherein the HA is preferably H1; and/or
(b) influenza B virus HA.

6. The method of any preceding claim, wherein HA in the sample is from an influenza virion.

7. The method of any preceding claim, wherein the sample includes (i) influenza virus HA and (ii) influenza virus antigens PB1, PB2, PA, NP, NA, M1, M2, NS1 and/or NS2.

8. The method of any preceding claim, wherein the sample includes:
(a) whole influenza virions;
(b) split influenza virions;
(c) purified surface antigens from influenza virions; and/or
(d) no viral proteins except for influenza virus proteins.

9. The method of any preceding claim, wherein the sample is:
(a) free from (i) serum components, (ii) egg proteins, and/or (iii) chicken DNA; and/or
(b) a bulk vaccine.

10. The method of any preceding claim, wherein the filtrate from step (i) is treated with detergent prior to RP-HPLC in step (ii).

11. A method for analyzing a vaccine comprising steps of: (a) purifying HA in the vaccine, or in a sample thereof, by the method of any preceding claim; and (b) using the results of step (a) to calculate the HA concentration in the vaccine.

12. A method for providing a bulk vaccine with a desired HA concentration, comprising steps of: (a) purifying HA in the vaccine, or in a sample thereof, by the method of any of claims 1-10; (b) using the results of step (a) to calculate the HA concentration in the bulk vaccine; and (c) using the results of step (b) to dilute the bulk vaccine to give the desired HA concentration; for example, wherein the desired HA concentration is between 1 and 150µg/ml *e.g*. 90µg/ml, 45µg/ml, 30 µg/ml, 15 µg/ml, 10 µg/ml, 7.5 µg/ml, 5 µg/ml, 3.8 µg/ml, 3.75 µg/ml, 1.9 µg/ml, or 1.5 µg/ml.

13. A method for providing a vaccine for patient use, comprising steps of: preparing a bulk vaccine with a desired HA content by the method of claim 12; and then extracting one or more unit doses of vaccine from the diluted bulk.

14. The method of claim 13, wherein an extracted unit dose is packaged as a kit component in combination with a second kit component, wherein the second kit component is a vaccine adjuvant.

15. A method for providing a bulk adjuvanted vaccine comprising steps of: preparing a bulk vaccine with a desired HA content by the method of claim 12; and then mixing the diluted bulk vaccine with an adjuvant.

16. A method for providing an adjuvanted vaccine for patient use, comprising steps of: providing a bulk adjuvanted vaccine by the method of claim 15; and then extracting one or more unit doses of vaccine from the bulk.

## Patentansprüche

1. Verfahren zur Aufreinigung von Influenzavirus-HA in einer Probe, umfassend die Schritte: (i) Ultrafiltration der Probe unter Verwendung einer Membran, die jegliches denaturiertes oder aggregiertes HA zurückhält, so dass man ein Filtrat erhält; und (ii) RP-HPLC des Filtrats, so dass jegliches darin vorhandenes HA von allen anderen darin vorhandenen Komponenten getrennt wird.

2. Verfahren nach Anspruch 1, wobei in Schritt (i) eine Ultrafiltrationsmembran mit einer Ausschlussgrenze von 300 kDa verwendet wird.

3. Verfahren nach einem vorhergehenden Anspruch, wobei in Schritt (ii) ein RP-HPLC-Träger von Polystyroldivinylbenzol-Partikeln mit einer Porengröße zwischen 500-5000 Å verwendet wird.

4. Verfahren nach einem vorhergehenden Anspruch, wobei die Probe HA von nur einem Influenzavirusstamm enthält.

5. Verfahren nach einem vorhergehenden Anspruch, wobei das HA Folgendes umfasst:
a. Influenza-A-Virus-HA, wobei es sich zum Beispiel bei dem HA um H1-, H3- oder H5-HA und vorzugsweise um H1 handelt; und/oder
b. Influenza-B-Virus-HA.

6. Verfahren nach einem vorhergehenden Anspruch, wobei das HA in der Probe von einem Influenzavirion stammt.

7. Verfahren nach einem vorhergehenden Anspruch, wobei die Probe (i) Influenzavirus-HA und (ii) Influenzavirus-Antigene PB1, PB2, PA, NP, NA, M1, M2, NS1 und/oder NS2 enthält.

8. Verfahren nach einem vorhergehenden Anspruch, wobei die Probe Folgendes enthält:
a. intakte Influenzavirionen;
b. gespaltene Influenzavirionen;
c. aufgereinigte Oberflächenantigene von Influenzavirionen; und/oder
d. keine Virusproteine außer Influenzavirusproteine.

9. Verfahren nach einem vorhergehenden Anspruch, wobei die Probe:
a. frei von (i) Serumkomponenten, (ii) Eiproteinen und/oder (iii) Hühner-DNA ist; und/oder
b. eine Impfstoffmasse ist.

10. Verfahren nach einem vorhergehenden Anspruch, wobei das Filtrat aus Schritt (i) vor RP-HPLC in Schritt (ii) mit Detergens versetzt wird.

11. Verfahren zur Analyse eines Impfstoffs, umfassend die Schritte: (a) Aufreinigen von HA in dem Impfstoff, oder in einer Probe davon, mit dem Verfahren nach einem vorhergehenden Anspruch; und (b) Verwenden der Ergebnisse aus Schritt (a) zur Berechnung der HA-Konzentration im Impfstoff.

12. Verfahren zur Bereitstellung einer Impfstoffmasse mit einer gewünschten HA-Konzentration, umfassend die Schritte: (a) Aufreinigen von HA in dem Impfstoff, oder in einer Probe davon, mit dem Verfahren nach einem der Ansprüche 1-10; (b) Verwenden der Ergebnisse aus Schritt (a) zur Berechnung der HA-Konzentration in der Impfstoffmasse; und (c) Verwenden der Ergebnisse aus Schritt (b) zum Verdünnen der Impfstoffmasse, so dass sich die gewünschte HA-Konzentration ergibt; wobei zum Beispiel die gewünschte HA-Konzentration zwischen 1 und 150 µg/ml, z.B. bei 90 µg/ml, 45 µg/ml, 30 µg/ml, 15 µg/ml, 10 µg/ml, 7,5 µg/ml, 5 µg/ml, 3,8 µg/ml, 3,75 µg/ml, 1,9 µg/ml oder 1,5 µg/ml liegt.

13. Verfahren zur Bereitstellung eines Impfstoffs zur Patientenanwendung, umfassend die Schritte: Herstellen einer Impfstoffmasse mit einem gewünschten HA-Gehalt mit dem Verfahren nach Anspruch 12; und anschließendes Extrahieren einer oder mehrerer Impfstoffdosiseinheiten aus der verdünnten Masse.

14. Verfahren nach Anspruch 13, wobei eine extrahierte Dosiseinheit als Kitkomponente in Kombination mit einer zweiten Kitkomponente abgepackt wird, wobei es sich bei der zweiten Kitkomponente um ein Impfstoffadjuvans handelt.

15. Verfahren zur Bereitstellung einer mit Adjuvans versehenen Impfstoffmasse, umfassend die Schritte: Herstellen einer Impfstoffmasse mit einem gewünschten HA-Gehalt mit dem Verfahren nach Anspruch 12; und anschließendes Mischen der verdünnten Impfstoffmasse mit einem Adjuvans.

16. Verfahren zur Bereitstellung eines mit Adjuvans versehenen Impfstoffs zur Patientenanwendung, umfassend die Schritte: Bereitstellen einer mit Adjuvans versehenen Impfstoffmasse mit dem Verfahren nach Anspruch 15; und anschließendes Extrahieren einer oder mehrerer Impfstoffdosiseinheiten aus der Masse.

## Revendications

1. Méthode de purification de l'HA du virus de la grippe dans un échantillon, comprenant les étapes constituées par : (i) l'ultrafiltration de l'échantillon à l'aide d'une membrane qui retient toute HA dénaturée ou agrégée pour donner un filtrat ; et (ii) RP-HPLC du filtrat pour séparer toute HA dans celui-ci de tout autre composant dans celui-ci.

2. Méthode selon la revendication 1, **caractérisée en ce que** l'étape (i) utilise une membrane d'ultrafiltration ayant un seuil de coupure de 300 kDa.

3. Méthode selon l'une ou l'autre des revendications précédentes, **caractérisée en ce que** l'étape (ii) utilise un support RP-HPLC de particules de polystyrènedivinylbenzène ayant une taille de pore comprise entre 500 et 5000 Å.

4. Méthode selon l'une quelconque des revendications précédentes, **caractérisée en ce que** l'échantillon contient l'HA d'une souche de virus de la grippe uniquement.

5. Méthode selon l'une quelconque des revendications précédentes, **caractérisée en ce que** l'HA comprend :
(a) l'HA du virus de la grippe A, par exemple l'HA est H1, H3 ou H5, et l'HA étant de préférence H1 ; et/ou
(b) l'HA du virus de la grippe B.

6. Méthode selon l'une quelconque des revendications précédentes, **caractérisée en ce que** l'HA dans l'échantillon provient d'un virion de la grippe.

7. Méthode selon l'une quelconque des revendications précédentes, **caractérisée en ce que** l'échantillon comprend (i) l'HA du virus de la grippe et (ii) les antigènes PB1, PB2, PA, NP, NA, M1, M2, NS1 et/ou NS2 du virus de la grippe.

8. Méthode selon l'une quelconque des revendications précédentes, **caractérisée en ce que** l'échantillon comprend :
(a) des virions de la grippe entiers ;
(b) des virions de la grippe fragmentés ;
(c) des antigènes de surface purifiés de virions de la grippe ; et/ou
(d) aucune protéine virale sauf des protéines du virus de la grippe.

9. Méthode selon l'une quelconque des revendications précédentes, **caractérisée en ce que** l'échantillon est :
(a) dépourvu (i) de composants sériques, (ii) de protéines de l'oeuf, et/ou (iii) d'ADN de poulet ; et/ou
(b) un vaccin en vrac.

10. Méthode selon l'une quelconque des revendications précédentes, **caractérisée en ce que** le filtrat de l'étape (i) est traité avec du détergent avant RP-HPLC dans l'étape (ii).

11. Méthode d'analyse d'un vaccin comprenant les étapes consistant à : (a) purifier l'HA dans le vaccin, ou dans un échantillon de celle-ci, par la méthode selon l'une quelconque des revendications précédentes ; et (b) utiliser les résultats de l'étape (a) pour calculer la concentration d'HA dans le vaccin.

12. Méthode d'obtention d'un vaccin en vrac ayant une concentration d'HA désirée, comprenant les étapes consistant à : (a) purifier l'HA dans le vaccin, ou dans un échantillon de celle-ci, par la méthode selon l'une quelconque des revendications 1 à 10 ; (b) utiliser les résultats de l'étape (a) pour calculer la concentration d'HA dans le vaccin en vrac ; et (c) utiliser les résultats de l'étape (b) pour diluer le vaccin en vrac pour donner la concentration d'HA désirée, par exemple, la concentration d'HA désirée étant comprise entre 1 et 150 µg/ml, par exemple 90 µg/ml, 45 µg/ml, 30 µg/ml, 15 µg/ml, 10 µg/ml, 7,5 µg/ml, 5 µg/ml, 3,8 µg/ml, 3,75 µg/ml, 1,9 µg/ml, ou 1,5 µg/ml.

13. Méthode d'obtention d'un vaccin à utiliser par un patient, comprenant les étapes consistant à :
préparer un vaccin en vrac avec une teneur en HA désirée par la méthode selon la revendication 12 ; et ensuite extraire une ou plusieurs doses unitaires de vaccin à partir du vaccin en vrac dilué.

14. Méthode selon la revendication 13, **caractérisée en ce qu'**une dose unitaire extraite est conditionnée sous forme d'un composant du kit en combinaison avec un deuxième composant du kit, le deuxième composant du kit étant un adjuvant de vaccin.

15. Méthode d'obtention d'un vaccin adjuvé en vrac comprenant les étapes qui consistent à : préparer un vaccin en vrac avec une teneur en HA désirée par la méthode selon la revendication 12 ; et ensuite mélanger avec un adjuvant le vaccin en vrac dilué.

16. Méthode d'obtention d'un vaccin adjuvé destiné à l'usage d'un patient, comprenant les étapes consistant à : obtenir un vaccin adjuvé en vrac par la méthode selon la revendication 15 ; et ensuite extraire une ou plusieurs doses unitaires de vaccin à partir du vaccin en vrac.


### SEQUENCE LISTING

<110> NOVARTIS AG
   DORMITZER Philip
   RINELLA Paola
   WEN Yingxia
   wu Ping
   PALMER Gene
<120> ASSAYS FOR INFLUENZA VIRUS HEMAGGLUTININS
<130> P054582WO
<140> PCT/IB2010/.........
   <141> 2010-05-28
<150> US61/217,405
   <151> 2009-05-29
<160> 2
<170> SeqWin99, version 1.02
<210> 1
   <211> 326
   <212> PRT
   <213> Influenza A virus
<400> 1
   Asp Thr Leu Cys Ile Gly Tyr His Ala Asn Asn Ser Thr Asp Thr Val
   1               5                   10                  15
   Asp Thr Val Leu Glu Lys Asn Val Thr Val Thr His Ser Val Asn Leu
               20                  25                  30
   Leu Glu Asp Lys His Asn Gly Lys Leu Cys Lys Leu Arg Gly Val Ala
           35                  40                  45
   Pro Leu His Leu Gly Lys Cys Asn Ile Ala Gly Trp Ile Leu Gly Asn
       50                  55                  60
   Pro Glu Cys Glu Ser Leu Ser Thr Ala Ser Ser Trp Ser Tyr Ile Val
   65                  70                  75                  80
   Glu Thr Pro Ser Ser Asp Asn Gly Thr Cys Tyr Pro Gly Asp Phe Ile
                   85                  90                  95
   Asp Tyr Glu Glu Leu Arg Glu Gln Leu Ser Ser Val Ser Ser Phe Glu
               100                 105                 110
   Arg Phe Glu Ile Phe Pro Lys Thr Ser Ser Trp Pro Asn His Asp Ser
           115                 120                 125
   Asn Lys Gly Val Thr Ala Ala Cys Pro His Ala Gly Ala Lys Ser Phe
       130                 135                 140
   Tyr Lys Asn Leu Ile Trp Leu Val Lys Lys Gly Asn Ser Tyr Pro Lys
   145                 150                 155                 160
   Leu Ser Lys Ser Tyr Ile Asn Asp Lys Gly Lys Glu Val Leu Val Leu
                   165                 170                 175
   Trp Gly Ile His His Pro Ser Thr Ser Ala Asp Gln Gln Ser Leu Tyr
               180                 185                 190
   Gln Asn Ala Asp Thr Tyr Val Phe Val Gly Ser Ser Arg Tyr Ser Lys
           195                 200                 205
   Lys Phe Lys Pro Glu Ile Ala Ile Arg Pro Lys Val Arg Asp Gln Glu
       210                 215                 220
   Gly Arg Met Asn Tyr Tyr Trp Thr Leu Val Glu Pro Gly Asp Lys Ile
   225                 230                 235                 240
   Thr Phe Glu Ala Thr Gly Asn Leu Val Val Pro Arg Tyr Ala Phe Ala
                   245                 250                 255
   Met Glu Arg Asn Ala Gly Ser Gly Ile Ile Ile Ser Asp Thr Pro Val
               260                 265                 270
   His Asp Cys Asn Thr Thr Cys Gln Thr Pro Lys Gly Ala Ile Asn Thr
           275                 280                 285
   Ser Leu Pro Phe Gln Asn Ile His Pro Ile Thr Ile Gly Lys Cys Pro
       290                 295                 300
   Lys Tyr Val Lys Ser Thr Lys Leu Arg Leu Ala Thr Gly Leu Arg Asn
   305                 310                 315                 320
   Ile Pro Ser Ile Gln Ser
                   325
<210> 2
   <211> 326
   <212> PRT
   <213> Influenza A virus
<400> 2
   Asp Thr Ile Cys Ile Gly Tyr His Ala Asn Asn Ser Thr Asp Thr Val
   1 5 10 15
   Asp Thr Val Leu Glu Lys Asn Val Thr Val Thr His Ser Val Asn Leu
   20 25 30
   Leu Glu Asp Asn His Asn Gly Lys Leu Cys Lys Leu Lys Gly Ile Ala
   35 40 45
   Pro Leu Gln Leu Gly Lys Cys Ser Ile Ala Gly Trp Ile Leu Gly Asn
   50 55 60
   Pro Glu Cys Glu Ser Leu Phe Ser Lys Lys Ser Trp Ser Tyr Ile Ala
   65 70 75 80
   Glu Thr Pro Asn Ser Glu Asn Gly Thr Cys Tyr Pro Gly Tyr Phe Ala
   85 90 95
   Asp Tyr Glu Glu Leu Arg Glu Gln Leu Ser Ser Val Ser Ser Phe Glu
   100 105 110
   Arg Phe Glu Ile Phe Pro Lys Glu Ser Ser Trp Pro Lys His Asn Val
   115 120 125
   Thr Lys Gly Val Thr Ala Ala Cys Ser His Lys Gly Lys Ser Ser Phe
   130 135 140
   Tyr Arg Asn Leu Leu Trp Leu Thr Glu Lys Asn Gly Ser Tyr Pro Asn
   145 150 155 160
   Leu Ser Lys Ser Tyr Val Asn Asn Lys Glu Lys Glu Val Leu Val Leu
   165 170 175
   Trp Gly Val His His Pro Ser Asn Ile Glu Asp Gln Lys Thr Ile Tyr
   180 185 190
   Arg Lys Glu Asn Ala Tyr Val Ser Val Val Ser Ser His Tyr Asn Arg
   195 200 205
   Arg Phe Thr Pro Glu Ile Ala Lys Arg Pro Lys Val Arg Asn Gln Glu
   210 215 220
   Gly Arg Ile Asn Tyr Tyr Trp Thr Leu Leu Glu Pro Gly Asp Thr Ile
   225 230 235 240
   Ile Phe Glu Ala Asn Gly Asn Leu Ile Ala Pro Trp Tyr Ala Phe Ala
   245 250 255
   Leu Ser Arg Gly Phe Gly Ser Gly Ile Ile Thr Ser Asn Ala Ser Met
   260 265 270
   Asp Glu Cys Asp Ala Lys Cys Gln Thr Pro Gln Gly Ala Ile Asn Ser
   275 280 285
   Ser Leu Pro Phe Gln Asn Val His Pro Val Thr Ile Gly Glu Cys Pro
   290 295 300
   Lys Tyr Val Arg Ser Thr Lys Leu Arg Met Val Thr Gly Leu Arg Asn
   305 310 315 320
   Ile Pro Ser Ile Gln Ser
   325